# EUROPEAN PATENT SPECIFICATION

(11) **EP 3 720 517 B1**
(45) Date of publication and mention of the grant of the patent: **21.01.2026**
(21) Application number: 18819017.7
(22) Date of filing: 04.12.2018
(51) Int. Cl.: A61L 29/08, A61L 29/16, A61K 9/00, C08L 89/00

(54) **INTRACRANIAL DRUG DELIVERY MATERIALS AND METHODS**
INTRAKRANIELLE WIRKSTOFFFREISETZUNGSMATERIALIEN UND VERFAHREN
SUBSTANCES ET MÉTHODES D'ADMINISTRATION DE MÉDICAMENT INTRACRÂNIENNE

(30) Priority: 06.12.2017 US 201762595087 P
(43) Date of publication of application: 14.10.2020
(73) Proprietor: Biotronik AG, 8180 Bülach (CH)
(72) Inventor: BETTS, Ronald E., La Jolla California 92037 (US)
(74) Representative: WSL Patentanwälte Partnerschaft mbB
(86) International application number: PCT/EP2018/083512
(87) International publication number: WO 2019/110600

(56) References cited:
- EP-A1- 3 228 335
- WO-A1-00/67816
- WO-A1-2013/007273
- WO-A1-2013/182503
- US-A1- 2016 250 388
- US-A1- 2017 136 155
- B. TYLER ET AL: "Local delivery of rapamycin: a toxicity and efficacy study in an experimental malignant glioma model in rats", NEURO-ONCOLOGY, vol. 13, no. 7, 1 July 2011 (2011-07-01), US, pages 700 - 709, XP055564968, ISSN: 1522-8517, DOI: 10.1093/neuonc/nor050

## Description

### FIELD OF THE INVENTION

This application relates generally to the field of minimally invasive delivery of therapeutic agents. Specifically, the present invention provides for materials and methods directed to minimally invasive, targeted delivery of agents such as drugs, penetrants, blood barrier augmentation agents or other compounds to certain localized brain regions through the use of delivery balloon catheters.

### BACKGROUND OF THE INVENTION

The worldwide incidence rate in 2012 of primary malignant brain and other CNS tumors was 3.4 per 100,000 or an overall total of >250,000 individuals that year. The incidence rates were higher in more developed countries. (GLOBOCAN 2012 v1.0, Cancer Incidence and Mortality Worldwide: IARC CancerBase No. 11 [Internet], International Agency for Research on Cancer; 2013). Glioblastoma multiforme (GBM) is the most common and most deadly primary brain tumor and accounts for 12% to 15% of all intracranial tumors (U.S. National Brain Tumor Society).

The incidence rate of childhood primary malignant and non-malignant brain and other CNS tumors in the U.S. is 5.5 per 100,000. (Ostrom QT, et al., CBTRUS Statistical Report: Primary Brain and Central Nervous System Tumors Diagnosed in the United States in 2009-*2013. Neuro Oncol.* 2016;18(sS):iv1-iv76).

Treatment of most primary brain tumors usually includes cancerous tissue debulking surgery, followed by radiation and chemotherapy to target any remaining and potentially invasive tumor cells. Post-surgery delivery of these treatments has presented significant challenges.

Chemotherapy has become a critical component to the treatment of primary brain tumors, as well as many other types of cancer. A prerequisite for the efficacy of any chemotherapeutic regimen is to ensure the drug reaches the tumor target in a therapeutically effective concentration.

Carmustine is a nonspecific alkylating agent and is frequently used for brain treatment after surgical debulking of tumors. Because carmustine can have considerable systemic side effects, local delivery is accomplished using carmustine releasing polyanhydride biodegradable wafers (Gliadel^{®} wafer) placed directly along the wall of the surgical cavity after the debulking procedure. Each wafer can deliver 7.7 mg carmustine while up to 8 wafers can be deposited.

The use of Gliadel^{®} wafers is, however, not without potential complications, as the physical presence of the wafer has been associated with difficulties during usual follow up magnetic resonance imaging activities (Colen, R.R. et al., World J. Radiol. 3(11), 2011, 266-272), as well as surgery-associated adverse events and localized toxicity (Chowdhary S. A., et al., J. Neurooncol 122, 2015, 367-382). In EP 3228335 A1, US 2016/0250388 A1, WO 2013/007273 A1 and US 2017/0136155 A1 balloon catheters with different coating are disclosed.

B. Tyler, B. et al., "Local delivery of rapamycin: a toxicity and efficacy study in an experimental malignant glioma model in rats", Neuro-Oncology, vol. 13, no. 7, 1 July 2011, 700-709, discloses Rapamycin administered intracranially for the treatment of gliomas, delivered from biodegradable caprolactone-glycolide (35:65) polymer beads.

WO 2013/182503 A1 discloses Rapamycin-40 esters in PLA coatings for balloon catheters for treatment of cancers.

Other drugs have been considered and the local delivery of sirolimus from biodegradable polymer beads when intracranially delivered into malignant glioma model rats has been reported (Tyler, B. et al., Neuro Oncology 13(7) 2011, 700-709). Sirolimus treated animals had significantly longer survival times as compared to control animals. Interestingly, radiation therapy in addition to the simultaneous sirolimus treatment led to longer survival duration than either treatment alone.

Another often utilized type of cancer therapy relates to delivery of effective amounts of radiation to the targeted tissue.

Delivery of radiation after surgery is commonly done and can be accomplished by external as well as internal (brachytherapy) means. Recently, GliaSite^{®}, a novel brachytherapy technique, has been approved by FDA for treatment of malignant brain tumors. The GliaSite^{®} device is an inflatable balloon catheter that is placed in the resection cavity after surgical removal of the tumor. The balloon is filled with an aqueous based radiation source and the device is allowed to remain for 3 to 6 days before removal. Frequently, Gliadel^{®} carmustine wafers are additionally placed in combination with the GliaSite^{®} catheter.

There is a need in the state of the art to improve the delivery methods and therapeutic endpoints in the field, as well as combining known drugs with lipophilic compounds in order to improve treatment paradigms for primary brain tumors.

### SUMMARY OF THE INVENTION

The present invention provides for a medical device suitable for intracranial delivery of therapeutic agents comprising a balloon catheter, wherein the balloon comprises a first coating and a second coating, wherein the second coating is applied on top of the first coating, wherein the second coating is comprised of a formulation having a first component and optionally a second component, characterized in that the first component of the formulation comprises a rapamycin 40-ester analog having the following structure: 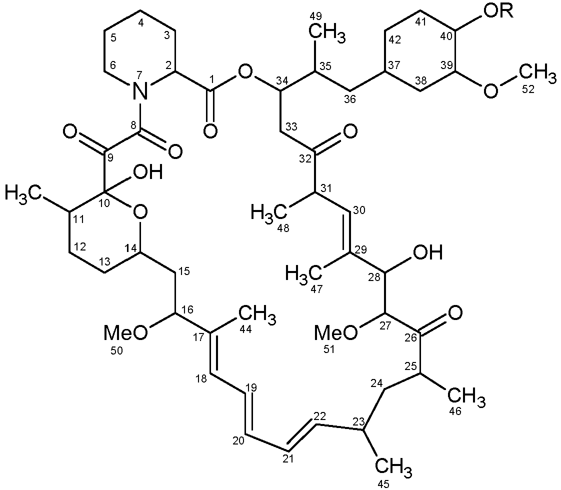 where R is C(O)-(CH₂)ₙ-X, n is 0, 1 or 2, X is a cyclic hydrocarbon having 3-9 carbons and optionally contains one or more unsaturated bonds, wherein C(O)-(CH₂)ₙ-X has one of the following structures: 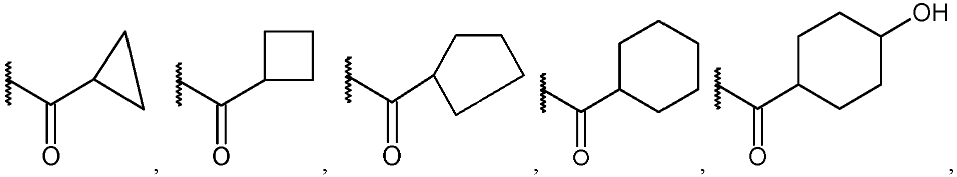 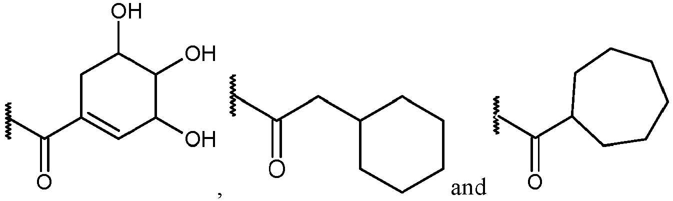 for use in treating primary brain tumor by intracranial delivery.

Preferably, the first coating comprises a water soluble polymer. Most preferably, the water soluble polymer is composed of a polymer or a protein having an approximate molecular weight of between 50 to 200 kD and preferably is a globular serum protein having an approximate molecular weight of between 65-70 kD. In a further embodiment the water soluble polymer is selected from blood proteins such as globulins and/or fibrinogens having molecular weights up to approximately 160 kD.

Preferably, the second coating comprises or consists of a therapeutic agent, wherein the therapeutic agent is selected from the group consisting of compounds as defined below. Optionally, a radiation solution is co-administered with the balloon catheter.

In another aspect, the present disclosure provides for a method of treating a primary brain cancer comprising:
(a) providing a balloon catheter comprising a first coating and a second coating, preferably a first coating and a second coating as defined herein, on the balloon's surface;
(b) accessing a subdural space in a skull of an individual;
(c) inserting the balloon catheter into the subdural space;
(d) inflating the balloon for an effective amount of time to allow for release of the second coating into the tissue.

In one aspect, the water soluble polymer is applied as the first coating, allowed to dry, then the second coating is applied.

The second coating is comprised of a formulation comprising a first component and optionally a second component. Preferably the first component is comprised of a lipophilic compound, wherein the lipophilic compound is a macrocyclic triene immunosuppressive compound having the structure: 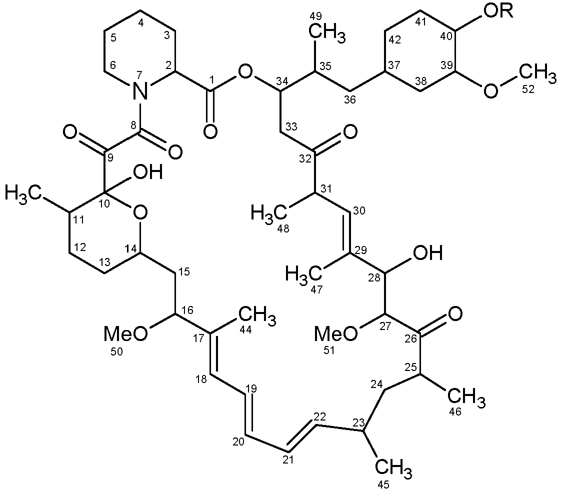 where R is C(O)-(CH₂)ₙ-X, n is 0, 1 or 2, X is a cyclic hydrocarbon having 3-8 carbons, optionally containing one or more unsaturated bonds, wherein C(O)-(CH₂)ₙ-X has one of the following structures:

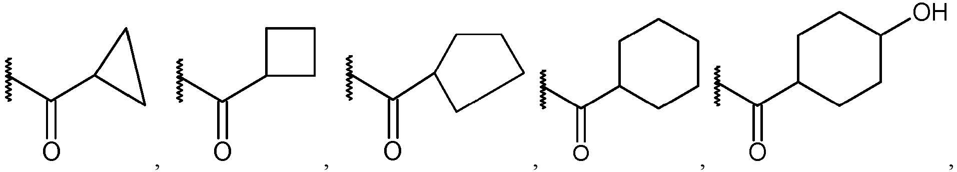

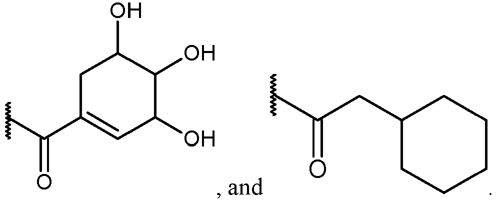

In an embodiment the first component consists of the rapamycin 40-ester analog having the structure: 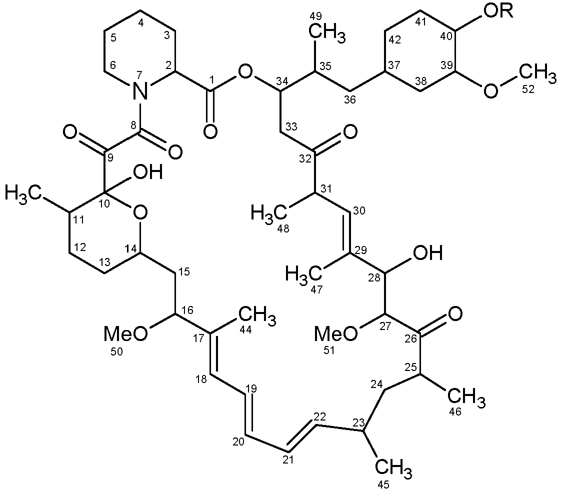 where R is C(O)-(CH₂)ₙ-X, n is 0, 1 or 2, X is a cyclic hydrocarbon having 3-9 carbons and optionally contains one or more unsaturated bonds, wherein C(O)-(CH₂)ₙ-X has one of the following structures:

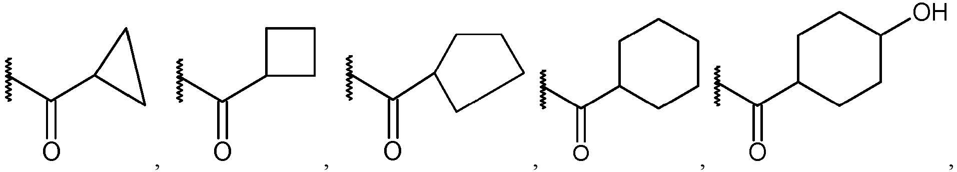

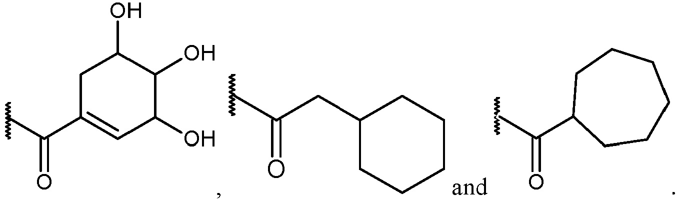

Preferably the optional second component comprises a fatty alcohol, fatty aldehyde or fatty acid. In another aspect the first component is comprised of only one of the above structures.

In still another aspect the first component is comprised of one of carmustine, temozolomide, lomustine, procarbazine or vincristine in combination with the macrocyclic triene immunosuppressive compound as defined herein. And in another aspect the first component is composed of one of carmustine, temozolomide, lomustine, procarbazine or vincristine and one of a macrocyclic triene immunosuppressive compound as defined herein.

In one aspect, the present disclosure teaches a method of manufacturing a medical device for intracranial delivery of therapeutic agents as a balloon catheter comprising: (a) providing a device, preferably a balloon catheter capable of radial expansion once inflated; (b) providing an aqueous solution of a water soluble polymer as defined herein preferably comprising from about 10% to about 30% of the water soluble polymer in a solution of water; (c) coating, preferably dip coating the device in the solution of (b); (d) allowing the coated device to dry; (e) applying a solution comprising the first and optionally the second component as defined herein to the device of (d); and (f) allowing the device of (e) to dry.

In one specific aspect, the present disclosure teaches a method of manufacturing a medical device for intracranial delivery of therapeutic agents as a balloon catheter comprising: (a) providing a balloon catheter capable of radial expansion once inflated; (b) providing an aqueous solution of a water soluble polymer preferably comprising from about 10% to about 30% of the water soluble polymer in a solution of water; (c) dip coating the balloon catheter in the solution of (b); (d) allowing the dip coated balloon catheter to dry; (e) applying a solution comprising a macrocyclic triene immunosuppressive compound and optionally at least one saturated fatty alcohol to the balloon catheter of (d); and (f) allowing the balloon catheter of (e) to dry.

### DETAILED DESCRIPTION OF THE INVENTION

As used herein, the term "macrocyclic triene immunosuppressive compound" includes rapamycin (sirolimus), everolimus, zotarolimus, biolimus, novolimus, myolimus, temsirolimus and the rapamycin derivatives described in this disclosure.

The present disclosure provides for devices and methods that can accommodate treatment of primary brain tumors in an individual. The medical devices of the present invention provide for a balloon catheter having a first coating and a second coating.
Preferably, the first coating is comprised of a water soluble material and forms the first coating layer on the surface of the medical device, preferably a balloon catheter. This first coating layer is preferably applied via dip coating, wherein the medical device, preferably the balloon catheter is placed into a solution comprising the water soluble polymer, which is applied to the surface of the medical device. Other suitable methods such as spraying or application of a solution by a thread, a needle, a cannula, a sponge or a piece of cloth can be used for the coating procedure. Following this application of the first coating, the medical device and preferably the balloon catheter is removed from the solution or the application device and allowed to dry, for example at ambient room temperature for a time less than 24 hours.

The water soluble polymer that is meant to comprise the bulk of the first coating is a polymer or a protein having an approximate molecular weight of between 50 to 200 kD. In one embodiment the water soluble polymer is selected from water soluble human serum proteins or water soluble blood proteins preferably having an approximate molecular weight of between 50 to 200 kD. In one embodiment the water soluble polymer is a polymer or a protein having an approximate molecular weight of between 65-70 kD, preferably a globular serum protein having an approximate molecular weight of between 65-70 kD. In a further embodiment the water soluble polymer is selected from blood proteins such as globulins and/or fibrinogens having molecular weights up to approximately 160 kD. More preferably, the water soluble polymer is human fibrinogen or immunoglobulin. Most preferably, the water soluble polymer is a human serum protein having at least 90% identity to the following sequence: 





 





 (SEQ ID NO: 1)
 In a most preferred embodiment of the invention the water soluble polymer is human serum albumin.

The second coating is comprised of a formulation further comprising a first component and optionally a second component. The first component of the formulation within the second coating comprises a rapamycin 40-ester analog having the following structure: 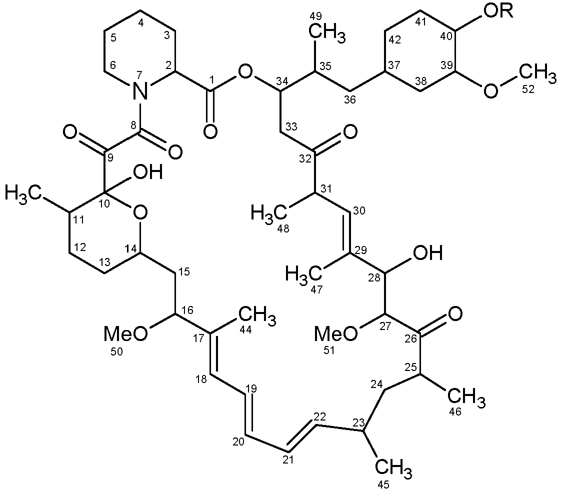 where R is C(O)-(CH₂)ₙ-X, n is 0, 1 or 2, X is a cyclic hydrocarbon having 3-9 carbons and optionally contains one or more unsaturated bonds, wherein C(O)-(CH₂)ₙ-X has one of the following structures:

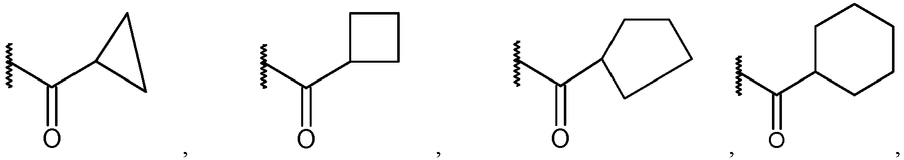

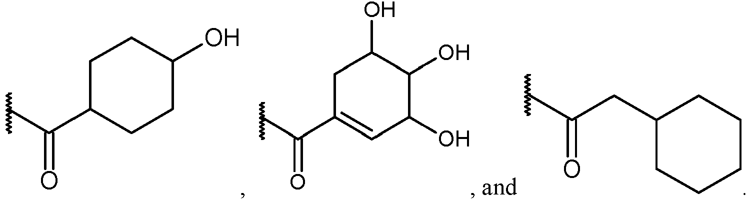

More preferably, the first component of the formulation within the second coating comprises a rapamycin 40-ester analog having the following structure: 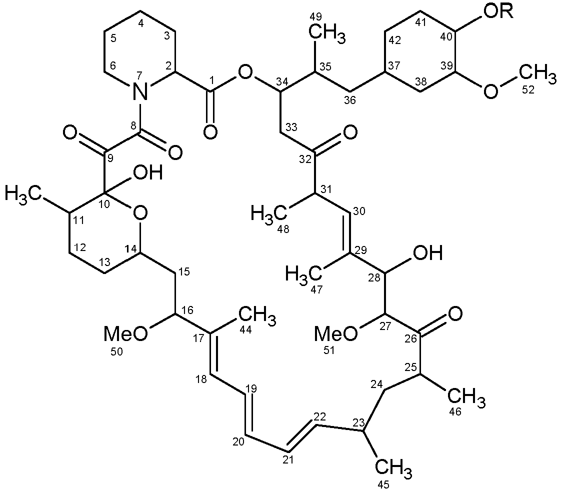 where R is C(O)-(CH₂)ₙ-X, n is 0, 1 or 2, X is a cyclic hydrocarbon having 3-8 carbons and optionally contains one or more unsaturated bonds, wherein C(O)-(CH₂)ₙ-X has one of the following structures: 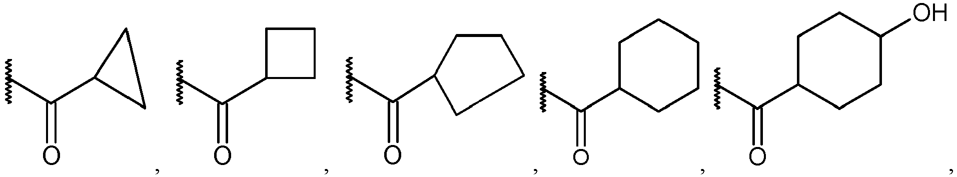 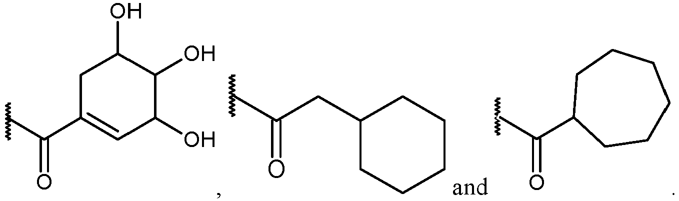 **In** embodiments, the optional second component of the second coating is a non-polymer, non-ionic, linear hydrocarbon or surfactant selected from the group consisting of a lipoic fatty alcohol or a fatty aldehyde or a fatty acid or combinations thereof. Preferably, the second coating is a member selected from the group consisting of lauryl alcohol, undecyl alcohol, myristyl alcohol, pentadecyl alcohol, palmitoleyl alcohol, palmityl alcohol, isocetyl alcohol, heptadecanol, lanolin alcohol, stearyl alcohol, isostearly alcohol, 12-hydroxystearyl alcohol, heneicosyl alcohol, behenyl alcohol, erucyl alcohol, 1-tricosanol, lignoceryl alcohol, 1-pentacosanol, ceryl alcohol, 1-heptacosanol, montanyl alcohol, 1-nonacosanol, myricyl alcohol, 1-hentriacontanol, lacceryl alcohol, 1-tritriacontanol, geddyl alcohol, arachidic acid, behenic acid, lignoceric acid and cerotic acid and the like as well as the aldehyde version of each.

Preferably the fatty alcohol or fatty aldehyde or nonionic surfactant is linear and contains at least 12 carbon atoms. Alternatively, more preferably, the second component of the second coating comprises a compound having the formula CₓH_{y}O, wherein x is at least 16 and y is at least 26. Yet more preferably, the second component of the second coating comprises a compound having the formula CₓH_{y}O, wherein x is at least 18 and at the most 35, and y is at least 36 and at the most 72. **In** one further embodiment, the second component of the second coating comprises a compound having the formula CₓH_{y}O, wherein x is at least 16 and y is at least 26 and the compound is a non-polymer, linear, branched or cyclic, saturated or unsaturated fatty alcohol. Yet more preferably, the second component of the second coating comprises a compound having the formula CₓH_{y}O, wherein x is at least 15 and at the most 35, and y is at least 30 and at the most 72 and the compound is a non-polymer, linear, branched or cyclic, saturated or unsaturated fatty alcohol. **In** one preferred embodiment there is provided a medical device and preferably a balloon catheter preferably having a surface that lacks structural modification and a second coating layer consisting of a non-polymer, saturated or unsaturated fatty alcohol or saturated or unsaturated fatty aldehyde as described herein and at least one therapeutic agent as described herein. **In** one further preferred embodiment there is provided a medical device and preferably a balloon catheter preferably having a surface that lacks structural modification herein with a second coating layer consisting of a non-polymer, saturated or unsaturated fatty alcohol as described herein and at least one therapeutic agent as described herein.

**In** one embodiment, the formulation of the second coating layer comprises 100% by weight of the first component. **In** another embodiment the formulation of the second coating layer comprises at least 80% by weight of the first component as defined herein and at least 15% by weight of second component as defined herein. **In** a preferred embodiment of the invention, the formulation of the second coating layer comprises from 60 to 95% by weight of the first component as defined herein and 5 to 40% by weight of the second component as defined. The formulation may further comprise an adequate amount of a solubilizing agent, such as a suitable organic solvent, and particularly a nonpolar organic solvent to facilitate suitable application of the formulation such as spray coating. Similarly, the amount of the first component as defined herein applied per drug eluting device, and in particular per balloon catheter is between 5 µg to 25 mg, preferably from about 1 mg to about 10 mg, depending on implant size. The amount of the second component is between 1 µg to 16.7 mg, preferably from about 2 µg to about 2.9 mg. **In** a most preferred embodiment, the drug load of the drug as defined herein per unit length of the catheter balloon from about 0,5 µg/mm² to 10 µg/mm² and preferably from about 1 to 3 µg/mm^{2.}.

**In** a preferred embodiment, the second coating comprising the first component and the second component is applied to the surface of the balloon after the first coating has dried. **In a** preferred embodiment the second coating is applied via spray coating on top of the first coating. In one aspect, the second coating is vacuum dried at a temperature higher than ambient room temperature, preferably in the range of 30 to 50 °C and most preferably at 40 °C.

Additionally, the present disclosure provides for a method of treating an individual having a primary brain tumor, the method comprising:
(a) providing a balloon catheter comprising a first coating and a second coating on the balloon's surface;
(b) accessing a subdural space in a skull of an individual;
(c) inserting the balloon catheter into the subdural space;
(d) inflating the balloon for an effective amount of time to allow for the release of the second coating into a tissue.

### Examples

The macrocyclic triene immunosuppressive compound of the present invention has more than one embodiment and may be described as comprising at least one of the following species from Table 1:

**Table 1**

| Description of CRC-015 species | | |
|---|---|---|
| Main structure | R is C(O)-(CH2)n-X having one of the following structures | Species |
| 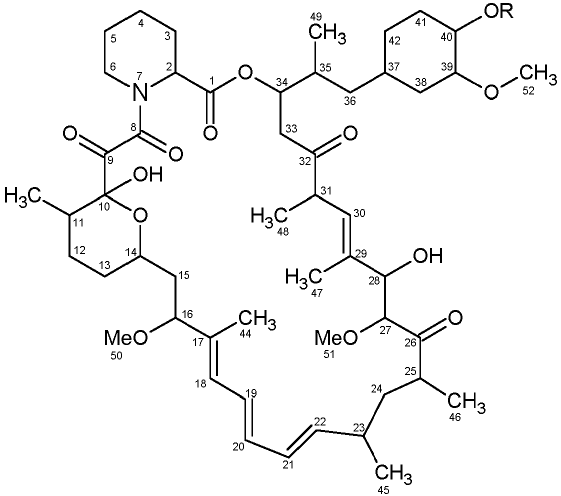 | 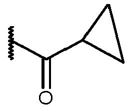 | CRC-015a |
| | 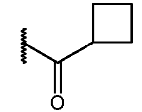 | CRC-015b |
| | 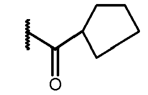 | CRC-015c |
| | 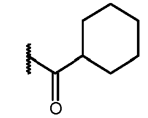 | CRC-015d |
| | 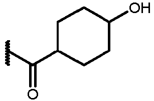 | CRC-015e |
| | 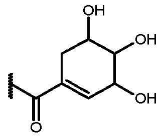 | CRC-015f |
| | 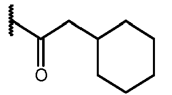 | CRC-015g |
| | 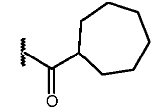 | CRC-015h |

CRC-015 is a term meant to encompass a genus and used to refer to each of the following species from Table 1: CRC-015a, CRC-015b, CRC-015c, CRC-015d, CRC-015e, CRC-015f, CRC-015g and CRC-015h.

### I. GliaSite^{®} Drug Delivery:

Drug delivery directly from the GliaSite^{®} balloon would potentially eliminate the use of drug delivery wafers. Drug could also be delivered from a separate balloon catheter used in conjunction with the conventional GliaSite^{®} device. As a proof of principle carmustine was delivered from a balloon catheter simulation of the GliaSite^{®} balloon.
A. PTCA 5 x 40mm (Biotronik^{®}) balloon catheters were first balloon expanded and dip coated using a 40% wt/vol solution of human serum albumin (Sigma A7736) (HSA) in D.I. water and allowed to dry overnight at ambient temperature (~21°C) to prepare the first coating. For the second coating, the HAS-coated balloons were deflated and 7 mg carmustine (Sigma CO400) dissolved in 50 micro.liter acetone was hand applied to each balloon using a 100 micro.liter glass syringe.
   Drug coated balloons were allowed to dry overnight at ambient temperature as before. The dried balloons were examined at 20X magnification which indicated a dull opaque coating appearance as compared to shiny uncoated balloons. For drug release testing the balloons were each placed into 30 mL PBS buffer pH 7.4 at ambient temperature and inflated. After 60 minutes the balloons were removed from solution and allowed to dry for evaluation of drug transfer. Examination at 20x revealed a shiny balloon surface devoid of coating and comparable to uncoated control balloons indicating carmustine release had occurred. For combination brachytherapy and chemotherapy, a drug coated GliaSite^{®} balloon catheter would simply be utilized in a manner consistent with usual GliaSite^{®} directions for use.
B. PTCA 3.5 x 20mm (Biotronik^{®}) balloon catheters were first balloon expanded and dip coated using a 40% wt/vol solution of human serum albumin (HSA) in deionized water and dried overnight at ambient temperature to prepare the first coating. For the second coating, the HSA coated balloons were deflated and 40 micro.liter of a 50 milli.gram/milli.liter solution of CRC-015 in acetone was hand applied to the balloon before allowing the balloon to dry overnight at ambient temperature.
C. Balloons with drug releasing layer were prepared as in B above. Balloons were spray coated using an acetone solution containing a mixture of 12.5 milli.gram/milli.liter CRC-015 and 4.16 milli.gram/milli.liter stearyl alcohol (Sigma 8.07680.0100) resulting in a 2 milli.gram CRC-015 drug dose per balloon. When released into tissue from the balloon the drug/fatty alcohol blend serves to provide an *in situ* deposition for sustained drug elution.

### II. Burr Hole Balloon Catheter Drug Delivery in Porcine Model

Burr hole surgery is a procedure to produce a hole in the skull to access the subdural space for removal of blood clots or to insert catheters for fluid drainage. The usual hole size is 14 milli.meter in diameter and is made with a specialized electric or hand drill. The present invention provides for balloon technology capable of localized drug delivery in conjunction with conventional burr hole surgery or with burr hole surgery utilizing much smaller cranial hole diameters, thus making it possible to conveniently deliver accurate drug amounts to well defined subdural locations in need of therapy. After the procedure has been completed smaller holes may simply be left to heal with the scalp closed over the opening.

A 5/8 inch diameter cranial burr hole was prepared from each of two recently euthanized animals utilized for an unrelated medical procedure. 100 micro. liter sterile saline was placed into the burr hole cavity before insertion of a CRC-015 coated balloon catheter. The balloon was then inflated at a steady rate and held for 60 seconds. The balloon was then deflated and withdrawn.

Any residual drug was extracted from the balloon with acetonitrile before measurement by HPLC. Results are reported in Table 2 and indicate rapid and convenient drug release from balloon to tissue. It is anticipated that other drugs/excipients and drug combinations could be delivered either together or individually at the same or different locations with minimal cranial invasiveness. It is also anticipated that reduction of burr hole size as well as improved balloon-to-balloon quantitative release and precision can be accomplished with additional development activity.

**Table 2**

| Delivery of CRC-015 from 2 mg Drug Delivery Cranial Balloon | | |
|---|---|---|
| **Animal** | **Balloon Residual Drug** | **Percent Drug Released** |
| 1 | 179 | 91.0 |
| 2 | 373 | 81.3 |

The inventions illustratively described herein can suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the future shown and described or any portion thereof, and it is recognized that various modifications are possible within the scope of the invention claimed. Thus, it should be understood that although the present invention has been specifically disclosed by preferred embodiments and optional features, modification and variation of the inventions herein disclosed can be resorted by those skilled in the art, and that such modifications and variations are considered to be within the scope of the inventions disclosed herein. The inventions have been described broadly and generically herein. Each of the narrower species and subgeneric groupings falling within the scope of the generic disclosure also form part of these inventions. This includes the generic description of each invention with a proviso or negative limitation removing any subject matter from the genus, regardless of whether or not the excised materials specifically resided therein.

In addition, where features or aspects of an invention are described in terms of the Markush group, those schooled in the art will recognize that the invention is also thereby described in terms of any individual member or subgroup of members of the Markush group. It is also to be understood that the above description is intended to be illustrative and not restrictive. Many embodiments will be apparent to those of ordinary skill in the art upon reviewing the above description. The scope of the invention should therefore, be determined not with reference to the above description, but should instead be determined with reference to the appended claims, along with the full scope of equivalents to which such claims are entitled.

## Claims

1. A medical device suitable for intracranial delivery of therapeutic agents comprising a balloon catheter, wherein the balloon comprises a first coating and a second coating, wherein the second coating is applied on top of the first coating, wherein the second coating is comprised of a formulation having a first component and optionally a second component, **characterized in that** the first component of the formulation comprises a rapamycin 40-ester analog having the following structure: 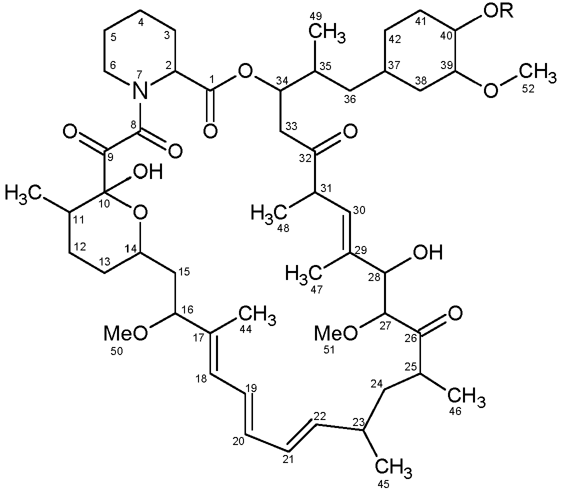
where R is C(O)-(CH₂)ₙ-X, n is 0, 1 or 2, X is a cyclic hydrocarbon having 3-9 carbons and optionally contains one or more unsaturated bonds, wherein C(O)-(CH₂)ₙ-X has one of the following structures: 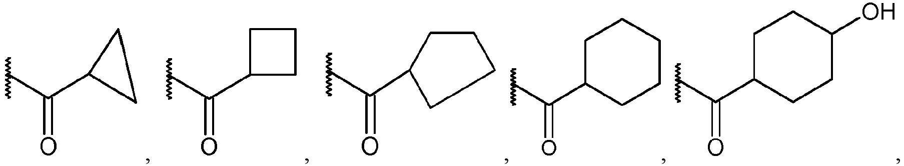 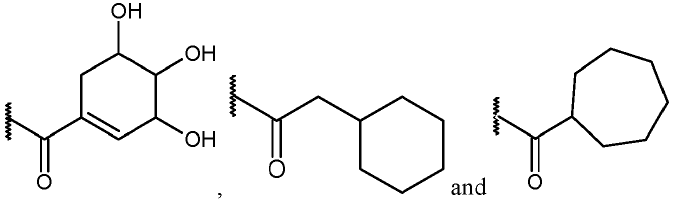
for use in treating primary brain tumor by intracranial delivery.

2. The medical device according to claim 1, wherein the first coating comprises at least one water soluble polymer.

3. The medical device according to claim 2, wherein the at least one water soluble polymer comprises a globular serum protein having an approximate molecular weight of between 65-70 kD.

4. The medical device according to any preceding claim, wherein the second component is a non-polymer, non-ionic, linear hydrocarbon or surfactant selected from the group consisting of a lipoic fatty alcohol or a fatty aldehyde or a fatty acid or combinations thereof.

5. The medical device according to claim 4, wherein a hydrocarbon or surfactant is a fatty alcohol having the formula CₓH_{y}O, wherein x is at least 18 and at the most 35, and y is at least 38 and at the most 72.

6. The medical device according to claims 1 to 3, wherein the second coating consists of the rapamycin 40-ester analog having the following structure: 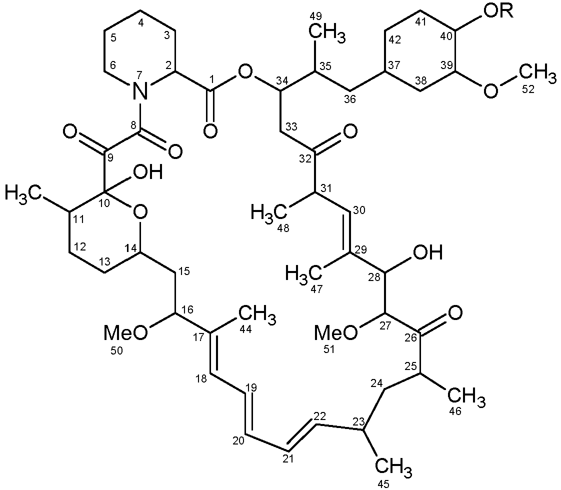 where R is C(O)-(CH₂)ₙ-X, n is 0, 1 or 2, X is a cyclic hydrocarbon having 3-9 carbons and optionally contains one or more unsaturated bonds, wherein C(O)-(CH₂)ₙ-X has one of the following structures:

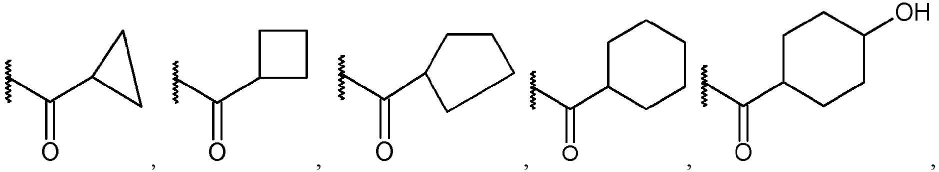

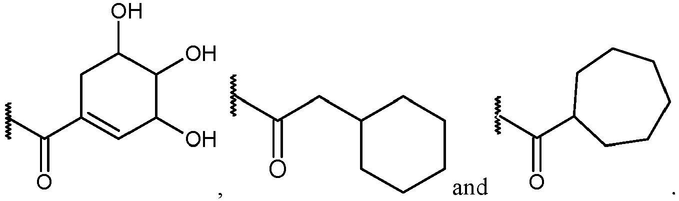

## Patentansprüche

1. Medizinische Vorrichtung, die zur intrakraniellen Verabreichung von Therapeutika geeignet ist, umfassend einen Ballonkatheter, wobei der Ballon eine erste Beschichtung und eine zweite Beschichtung umfasst, wobei die zweite Beschichtung auf die erste Beschichtung aufgebracht ist, wobei die zweite Beschichtung aus einer Formulierung mit einer ersten Komponente und optional einer zweiten Komponente besteht, **dadurch gekennzeichnet, dass** die erste Komponente der Formulierung ein Rapamycin-40-Ester-Analogon mit der folgenden Struktur umfasst: 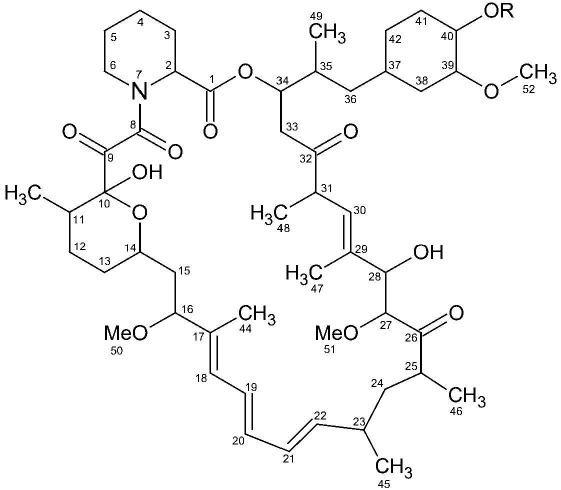
wobei R C(O)-(CH₂)ₙ-X ist, n 0, 1 oder 2 ist, X ein cyclischer Kohlenwasserstoff mit 3 bis 9 Kohlenstoffatomen ist und optional eine oder mehrere ungesättigte Bindungen enthält, wobei C(O)-(CH₂)ₙ-X eine der folgenden Strukturen aufweist: 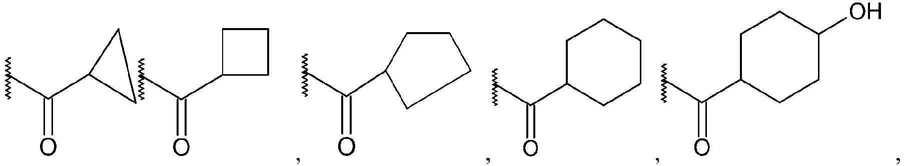 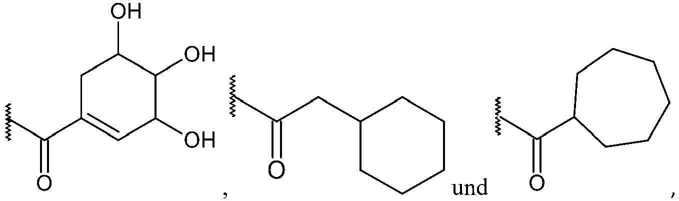
zur Verwendung bei der Behandlung von primären Hirntumoren durch intrakranielle Verabreichung.

2. Medizinische Vorrichtung gemäß Anspruch 1, wobei die erste Beschichtung mindestens ein wasserlösliches Polymer umfasst.

3. Medizinische Vorrichtung gemäß Anspruch 2, wobei das mindestens eine wasserlösliche Polymer ein globuläres Serumprotein mit einem ungefähren Molekulargewicht zwischen 65 und 70 kD umfasst.

4. Medizinische Vorrichtung nach einem der vorstehenden Ansprüche, wobei die zweite Komponente ein nicht-polymerer, nicht-ionischer, linearer Kohlenwasserstoff oder ein Tensid ist, ausgewählt aus der Gruppe bestehend aus einem von Liponsäure abgeleiteten Fettalkohol oder einem Fettsäurealdehyd oder einer Fettsäure oder Kombinationen davon.

5. Medizinische Vorrichtung nach Anspruch 4, wobei ein Kohlenwasserstoff oder Tensid ein Fettalkohol mit der Formel CₓH_{y}O ist, wobei x mindestens 18 und höchstens 35 ist und y mindestens 38 und höchstens 72 ist.

6. Medizinische Vorrichtung nach den Ansprüchen 1 bis 3, wobei die zweite Beschichtung aus dem Rapamycin-40-Ester-Analogon mit der folgenden Struktur besteht: 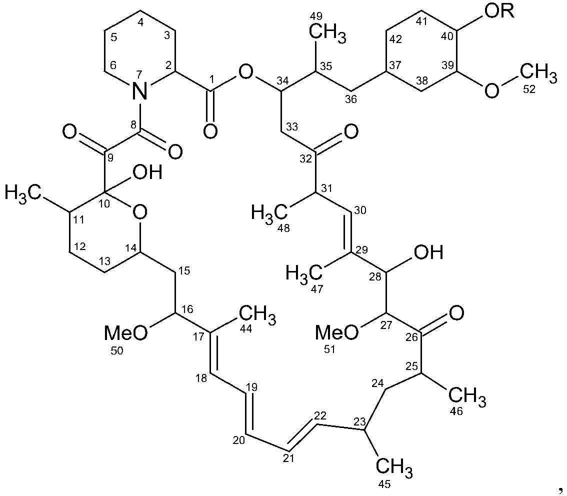 wobei R C(O)-(CH₂)ₙ-X ist, n 0, 1 oder 2 ist, X ein cyclischer Kohlenwasserstoff mit 3 bis 9 Kohlenstoffatomen ist und optional eine oder mehrere ungesättigte Bindungen enthält, wobei C(O)-(CH₂)ₙ-X eine der folgenden Strukturen aufweist:

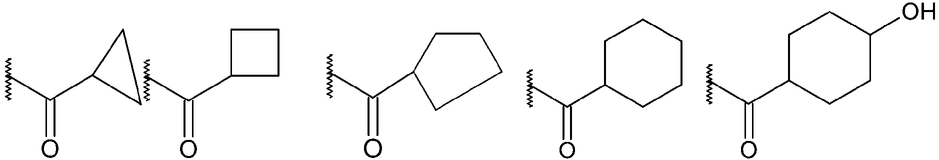

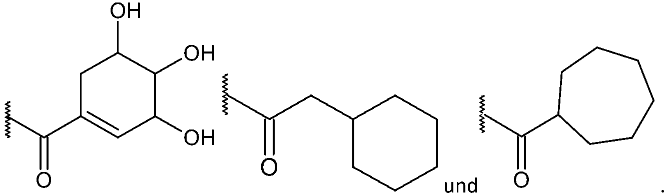

## Revendications

1. Dispositif médical adapté à l'administration intracrânienne d'agents thérapeutiques, comprenant un cathéter à ballonnet, dans lequel le ballonnet comprend un premier revêtement et un deuxième revêtement, le deuxième revêtement étant appliqué sur le premier revêtement, le deuxième revêtement étant constitué d'une formulation ayant un premier composant et éventuellement un deuxième composant, **caractérisé en ce que** le premier composant de la formulation comprend un analogue 40-ester de la rapamycine ayant la structure suivante : 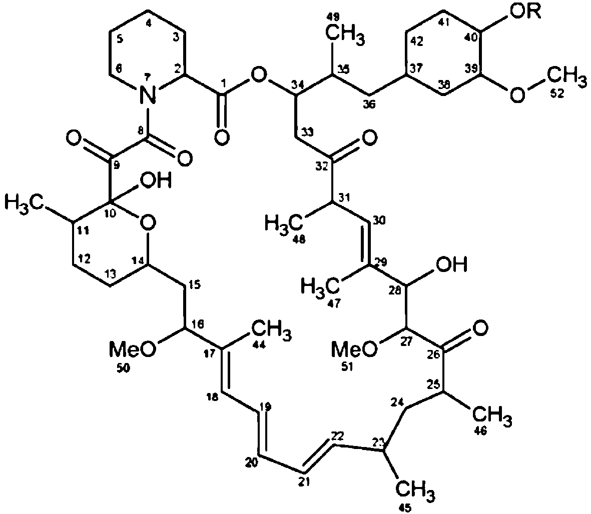
où R représente C(O)-(CH₂)ₙ-X, n est égal à 0, 1 ou 2, X est un hydrocarbure cyclique ayant 3-9 atomes de carbone et contient facultativement une ou plusieurs liaisons insaturées, C(O)-(CH₂)ₙ-X ayant l'une des structures suivantes : 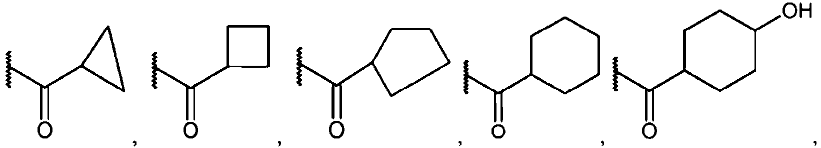 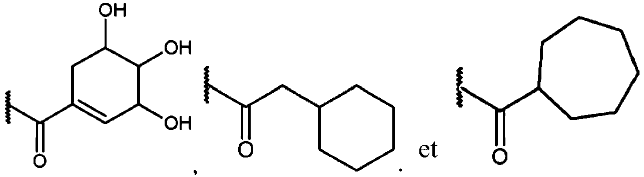
destiné au traitement des tumeurs cérébrales primaires par administration intracrânienne.

2. Dispositif médical selon la revendication 1, dans lequel le premier revêtement comprend au moins un polymère hydrosoluble.

3. Dispositif médical selon la revendication 2, dans lequel ledit au moins un polymère hydrosoluble comprend une protéine sérique globulaire ayant un poids moléculaire approximatif compris entre 65 et 70 kD.

4. Dispositif médical selon l'une quelconque des revendications précédentes, dans lequel le deuxième composant est un hydrocarbure linéaire non ionique, non polymère ou un tensioactif choisi dans le groupe constitué par un alcool gras lipoïque ou un aldéhyde gras ou un acide gras ou des combinaisons de ceux-ci.

5. Dispositif médical selon la revendication 4, dans lequel un hydrocarbure ou un tensioactif est un alcool gras répondant à la formule CₓH_{y}O, dans laquelle x est au moins égal à 18 et au plus égal à 35, et y est au moins égal à 38 et au plus égal à 72.

6. Dispositif médical selon les revendications 1 à 3, dans lequel le deuxième revêtement est constitué de l'analogue 40-ester de la rapamycine ayant la structure suivante : 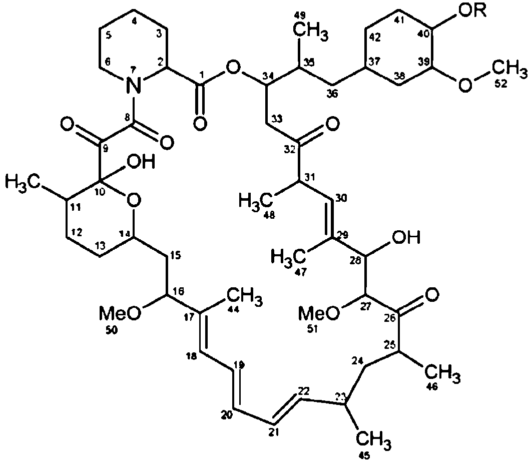 où R représente C(O)-(CH₂)ₙ-X, n est égal à 0, 1 ou 2, X est un hydrocarbure cyclique ayant 3-9 atomes de carbone et contient facultativement une ou plusieurs liaisons insaturées, C(O)-(CH₂)ₙ-X ayant l'une des structures suivantes :
